## Europäisches Patentamt
## European Patent Office
## Office européen des brevets

(19)

(11) Veröffentlichungsnummer : **0 149 443**
**B1**

(12)

# EUROPÄISCHE PATENTSCHRIFT

(45) Veröffentlichungstag der Patentschrift :
02.11.89

(51) Int. Cl.⁴ : **A 01 G  9/14, A 01 G  9/20**

(21) Anmeldenummer : **84890230.0**

(22) Anmeldetag : **26.11.84**

(54) **Klemmverbindung zwischen einer Doppelstegplatte und einem Profil, insbesondere bei Kleingewächshäusern.**

(30) Priorität : 25.11.83 AT 4139/83

(43) Veröffentlichungstag der Anmeldung :
24.07.85 Patentblatt 85/30

(45) Bekanntmachung des Hinweises auf die Patenterteilung : 02.11.89 Patentblatt 89/44

(84) Benannte Vertragsstaaten :
CH DE FR LI NL

(56) Entgegenhaltungen :
EP--A-- 0 052 188
CH--A--   555 134
DE--A-- 2 346 095
DE--A-- 2 632 040
DE--A-- 3 121 047
FR--A--    48 204
FR--A-- 1 490 551

(73) Patentinhaber : Wüster, Heinrich
Auwerk 18
A-6460 Imst/Tirol (AT)

(72) Erfinder : Wüster, Heinrich
Auwerk 18
A-6460 Imst/Tirol (AT)

(74) Vertreter : Berger, Erhard, Dr.
Siebensterngasse 39 Postfach 306
A-1071 Wien (AT)

**Beschreibung**

Die Erfindung betrifft ein Doppelstegplattenelement mit von Anschluß- bzw. Abschlußprofilen eingefaßten Rändern, insbesondere für Frühbeetkästen oder Kleingewächshäusern.

Insbesondere für Frühbeetkästen oder Kleingewächshäuser werden in zunehmenden Maße statt der bisher verwendeten Eindeckung mit Glasscheiben, die aufgrund ihres großen Gewichtes starke Tragkonstruktionen erfordern, vor allem aber eine extrem schlechte Wärmedämmung aufweisen, sogenannte Doppelstegplatten aus Polypropylen oder Polycarbonat verwendet. Die Doppelstegplatten bilden mit ihren beiden, von längsverlaufenden Stegen auf Abstand gehaltenen Deckplatten Luftkammern, die eine hohe Wärmeisolierung ergeben. Zur Erzielung eines möglichst geringen Gewichtes und zur Materialkostenersparnis werden diese Doppelstegplatten sehr dünnwandig erzeugt, wobei sich zwar einerseits durch die zellenförmige Struktur eine ausreichende Eigensteifigkeit ergibt, anderseits jedoch die einzelnen Kammerwände so flexibel sind, daß sich eine fixe Verbindung zwischen einzelnen Plattenelementen nur schwierig durchführen läßt.

Für den Aufbau eines kastenförmigen Behälters für ein Frühbeet oder Gewächshaus ist aber eine stabile Verbindung der einzelnen Plattenelemente unerläßlich. Auf der anderen Seite müssen die längsverlaufenden Luftkammern gegen Flugstaub und Regenwasser abgedichtet werden, soll nicht in kurzer Zeit sowohl die Transparenz als auch die Wärmedämmung erheblich verschlechtert werden.

Zur gegenseitigen Verbindung der einzelnen Plattenelemente und bzw. oder zur Abdichtung der Luftkammern werden auf die Ränder der Plattenelemente an ihren Stirnseiten und gegebenenfalls auch an ihren zu den Stegen parallelen Seiten Profile aufgebracht, die entweder Anschlußprofile, deren besondere Formgebung ein Aneinanderreihen beliebig vieler Plattenelemente in Form eines Baukastensystems und/oder eine Eck- oder Scharnierverbindung von Plattenelementen erlaubt, oder auch reine Anschlußprofile sein können. Allerdings hat es sich gezeigt, daß, bedingt durch die nur geringe Stabilität der Kammerwände der handelsüblichen Doppelstegplatten eine für die Gesamtstabilität der zu erstellenden Konstruktion entscheidende feste, auch auf Zug beanspruchbare Verbindung zwischen den Doppelstegplatten und diesen Profilen nur äußerst schwierig möglich ist.

Werden die Profile mit entsprechender Kraft auf die Ränder der Doppelstegplatte aufgepreßt, so tritt unweigerlich eine Deformation der Plattenstege ein, wodurch, insbesondere wenn es sich um eine Stirnseite der Platte handelt, die Verbindung unbrauchbar wird. Das gleiche Resultat ergibt sich auch, wenn zunächst die in diesem Fall aus Metall bestehenden Profile auf die Doppelstegplatte mehr oder weniger lose aufgeschoben werden, um erst nachher verpreßt zu werden.

Eine einigermaßen auch bei Zugbeanspruchung befriedigende Lösung ergäbe sich erst dann, wenn nach einem nicht zum Stand der Technik zählenden Vorschlag in die auf die Doppelstegplatte aufgeschobenen metallischen Profile in regelmäßigen Abständen kleine halbmondförmige Ausnehmungen ausgestanzt werden, wobei die sich ergebenden ausgestanzten, durchgedrückten Nasen Widerhaken bilden, die sich mit den Stegen der Platte verkeilen. Diese Lösung hat allerdings sowohl den Nachteil, daß diese Ausstanzungen ein Eindringen von Regenwasser und Verunreinigungen in die Plattenkammern zulassen, als auch daß diese Art von Ausstanzungen nur mit Metallprofilen realisierbar ist, die eine an sich schlechte Wärmedämmung aufweisen.

Aus der DE-A-3 121 047 ist eine in das Innere einer offenen Stirnseite einer Doppelstegplatte federnd einzusetzende U-förmige Abschlußleiste bekannt, die mit ihren beiden zu den Deckplatten der Doppelstegplatte parallelen Schenkeln in zueinander parallelen Schlitzen in den Stegenden eingesetzt ist und mit ihrer die beiden Schenkel verbindenden Basis mit der offenen Stirnseite der Doppelstegplatte bündig angeordnet ist und zwischen sich und den Deckplattenkanten Zwischenräume freiläßt, die mit einem Dichtungsmittel abgeschlossen werden müssen, wenn ein vollständiger Abschluß der offenen Stirnseite der Doppelstegplatte gewünscht wird. Diese jederzeit wieder entfernbare Abschlußleiste ist selbst als Feder ausgebildet, wobei zum Einsetzen die beiden Schenkel der Leiste etwas zueinandergedrückt, in die Schlitze eingeführt und dann ausgelassen werden, sodaß über die federnden Schenkel eine ausweichende Haftwirkung in den Schlitzen der Stege zustande kommt, um die Abschlußleiste gegen Herausfallen zu sichern. Bei dieser bekannten Abschlußleiste ist eine zugfeste Verbindung zwischen Leiste und Doppelstegplatte weder erwünscht noch möglich.

Aus der FR-A-48 204 ist eine Blechtüre mit zwei auf Distanz gehaltenen Blechtafeln als Füllung und Einfassungsprofilen als Randabschluß bekannt, wobei die Ränder der beiden Blechtafeln zu jeweils gegenüber der Blechtafelebene vertieften, nach außen offenen und im Querschnitt dreieckigen Rillen gebogen und zwischen den entsprechend geformten Kanälen der Distanzelemente und den entsprechend geformten, nach innen vorstehenden Randwülsten der Einfassungsprofile eingeklemmt sind. Bei der Herstellung des Türblattes werden die Einfassungsprofile in Rillenlängsrichtung auf die, in die Kanäle der Distanzelemente eingelegten Randrillen der Blechtafeln aufgeschoben, sodaß ein Formschluß zwischen Distanzelemente Blechtafel und Einfassungsprofil entsteht. Diese Verbindung ist nur bei vorgeformten steifen Randrillen ausführbar und daher für Doppelstegplatten aus hochtransparenten Kunststoff nicht anwendbar.

Aufgabe der Erfindung ist daher, die genannten Nachteile zu vermeiden und ein Doppelstegplattenelement mit von Anschluß- bzw. Abschlußprofilen eingefaßten Rändern, anzugeben, bei dem trotz der geringen Längs- und Quersteifigkeit von Doppelstegplatten eine auf Zug beanspruchbare Verbindung zwischen den Profilen und der Doppelstegplatte gewährleistet ist, und das die Verwendung von Profilen sowohl aus Metall als auch aus Kunststoff erlaubt.

Dies wird erfindungsgemäß durch die kennzeichnenden Merkmale des Anspruches 1 gelöst. Durch die erfindungsgemäße Ausbildung wird eine formschlüssige Verbindung zwischen den, den jeweiligen Rand der Doppelstegplatten umgreifenden Profilen und der Doppelstegplatte erreicht ohne letztere zu schwächen. Bei der vorliegenden Erfindung wird eine, je nach der Steifigkeit der verwendeten Doppelstegplatte, entsprechende Anzahl von Klemmkörpern eingesetzt und damit die Aufpreßkraft des jeweiligen Anschlußbzw. Abschlußprofiles variabel an die Doppelstegplatte angepaßt. Beim Aufpressen des Anschlußbzw. Abschlußprofiles wird dieses aufgeweitet, um nachher wieder einzufedern, wobei die Nasen des Profils über die Vorsprünge der Klemmkörper geschoben werden und zusammen mit den Deckplatten der Doppelstegplatte hinter den Vorsprüngen einrasten.

Bei einer Ausgestaltung der Erfindung gemäß Anspruch 2 wird jede Deckplatte jeweils zwischen einem Vorsprung eines Klemmkörpers und einer diesen hintergreifenden Nase des Anschluß- bzw. Abschlußprofiles eingeklemmt.

Bei einer Ausgestaltung der Erfindung gemäß Anspruch 3 wird die Doppelsteg-Hohlkammerplatte durch die eingesetzten Klemmkörper entsprechend aufgeweitet und an diesen Stellen verfestigt.

Eine besonders hohe Zugfestigkeit wird durch die Ausgestaltung gemäß Anspruch 4 erzielt werden.

Durch die Ausbildung der Klemmkörper gemäß Anspruch 5 wird der Zusammenbau des erfindungsgemäßen Doppelstegplattenelementes wesentlich erleichtert. Mit den Merkmalen des Anspruches 6 wird die Abstandhaltung der Klemmkörper beim Zusammenbau des erfindungsgemäßen Doppelstegplattenelementes vereinfacht.

Schließlich ermöglicht die Ausgestaltung der Erfindung gemäß Anspruch 7 eine einfache und kostengünstige Herstellung der Klemmkörper.

Bei dem erfindungsgemäßen Doppelstegplattenelement wird die Festigkeit der Doppelstegplatte durch die, in diese eingesetzten Klemmkörper örtlich erhöht.

Nachstehend ist die Erfindung anhand von Ausführungsbeispielen näher erläutert. Es zeigen :
Fig. 1 eine Schrägansicht auf eine bekannte Doppelstegplatte,
Fig. 2 einen Schnitt durch den Rand eines erfindungsgemäßen Doppelstegplattenelementes an einer Stirnseite der Doppelstegplatte,
Fig. 3 eine bevorzugte Ausführungsform des Klemmkörpers in Vorder- und Seitenansicht,

Fig. 4 in Seiten- und Stirnansicht eine Ausführungsform, bei der die einzelnen Klemmkörper auf einem Trägerkörper angeordnet sind,
Fig. 5 einen Schnitt längs der Linie V-V in Fig. 6 durch ein erfindungsgemäßes Doppelstegplattenelement an einer zu den Stegwänden parallelen Seite der Doppelstegplatte,
Fig. 6 einen Schnitt nach der Linie VI-VI in Fig. 5,
Fig. 7 schematisch die Aneinanderreihung zweier mit Anschlußprofilen versehener Doppelstegplattenelemente unter Verwendung eines Verbindungsprofils,
Fig. 8 schematisch eine Eck- bzw. Scharnierverbindung zweier mit Anschlußprofilen versehener Doppelstegplattenelemente, und
Fig. 9 ein Anschlußprofil zum direkten Aneinanderreihen zweier Doppelstegplattenelemente.

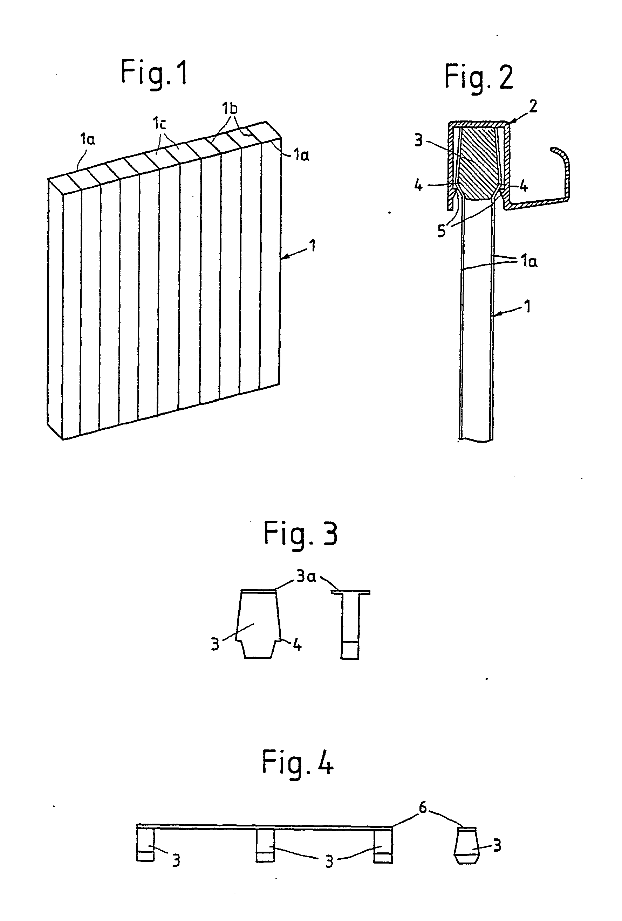

Eine Doppelstegplatte besteht, wie aus Fig. 1 ersichtlich ist, aus zwei Deckplatten 1a, die durch längsverlaufende Stegwände 1b auf Abstand gehalten sind. Die dadurch gebildeten längsverlaufenden Hohl- bzw Luftkammern 1c ergeben eine hohe Wärmedämmung der Doppelstegplatte 1. Unter den Stirnseiten der Doppelstegplatte versteht man jene Seiten, an denen die Luftkammern 1c ausmünden, während man bei den beiden anderen Seiten von den stegparallelen Seiten spricht.

In Fig. 2 ist nun ein erfindungsgemäßes Doppelstegplattenelement mit einem Anschlußprofil 2 an einer Stirnseite der Doppelstegplatte 1 dargestellt. Zur Verbindung von Doppelstegplatte 1 und Anschlußprofil 2 werden in regelmäßigen Abständen längs der Stirnseite der Doppelstegplatte 1 Klemmkörper 3 eingesteckt, die an ihren den Deckplatten 1a zugewendeten Seiten keilförmige Vorsprünge 4 aufweisen. Dadurch werden die dünnen Deckplatten 1a entsprechend der Keilform geringfügig verformt. Das mit der Doppelstegplatte 1 zu verbindende Profil 2 besitzt an seinen den Deckplatten 1a zugewendeten Seiten angeformte Nasen 5, die auf einer solchen Höhe vorgesehen sind, daß beim Aufschieben des Profils die Vorsprünge 4 der Klemmkörper 3 samt den darüberliegenden in diesem Bereich verformten Deckplatten 1a hinter diesen Nasen 5 einrasten, sodaß eine Klemmverbindung hoher Zugfestigkeit entsteht. Neben ihrer hohen Zugfestigkeit hat diese Klemmverbindung auch noch den Vorteil, daß sie problemlos fertigungsbedingte Dikkentoleranzen der Doppelstegplatte aufnehmen kann, da sich die flexiblen Deckplatten 1a den Klemmkörpern 3 anpassen und damit die Toleranz zu dem Profil 2 genau einhaltbar ist.

Fig. 3 zeigt eine Ausführungsform, bei der die Klemmkörper 3 an ihren oberen, dem Rand der Doppelstegplatte zugeordneten Enden mit Ansätzen 3a versehen sind, die, da sie beim Eindrücken der Klemmkörper in die Doppelstegplatte 1 zum Anliegen an den Stegen 1b letzterer kommen, einen Begrenzungsanschlag gegen ein zu weites Eindrücken der Klemmkörper bilden. Bei dieser Ausführungsform ist weiters die keilförmige Ausgestaltung der Vorsprünge 4 zu einer Sägezahn

form verstärkt, die eine noch bessere Einrastung mit dem Profil 2 gewährleistet. Praktische Versuche haben gezeigt, daß damit eine Verbindung zwischen Doppelstegplatte 1 und Profil 2 erreicht wird, deren Zugfestigkeit sogar über der der Doppelstegplatte selbst liegt.

Fig. 4 zeigt eine Ausführungsform, bei der die einzelnen Klemmkörper 3 auf einem bandförmigen Trägerkörper 6 angeordnet sind, der ihr gleichzeitiges Einbringen auf eine größere Länge der Doppelstegplatte erlaubt und eine zusätzliche Abdichtung gegen Feuchtigkeit ermöglicht.

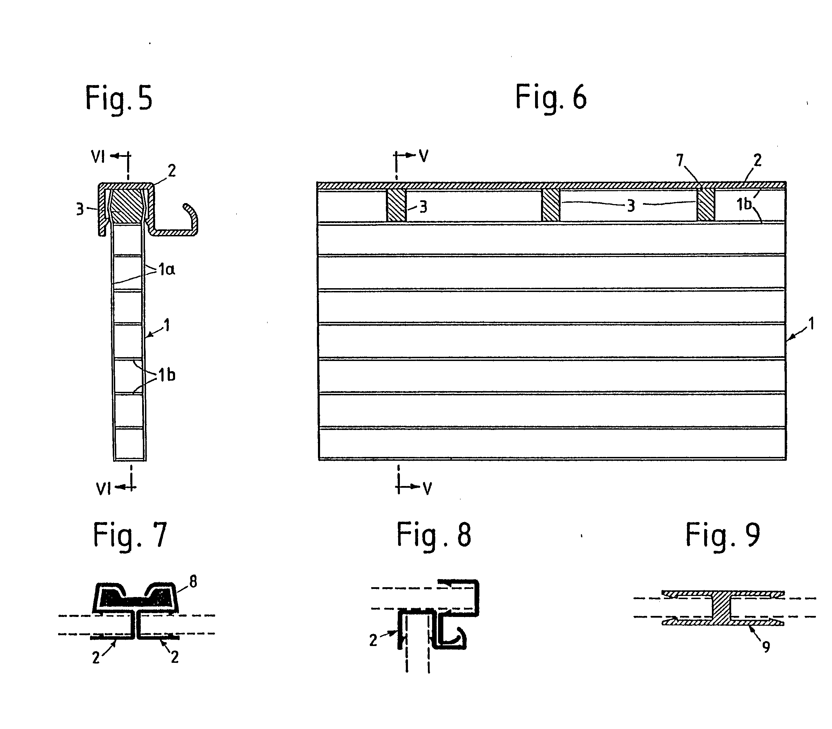

Die Fig. 5 und 6 zeigen ein erfindungsgemäßes Doppelstegplattenelement mit einem Anschlußprofil an einer stegparallelen Seite der Doppelstegplatte 1. Hier werden die Klemmkörper 3 in ausgestanzte Ausnehmungen 7 (Fig. 6) der obersten Stegwand 1b eingesetzt. Falls, wegen einer größeren Höhe der Klemmkörper 3 und der Profile 2, wie sie in den Fig. 2 bis 4 dargestellt ist, erforderlich, werden auch in der zweitobersten Stegwand derartige Ausnehmungen 7 vorgesehen.

Die Abstände zwischen den einzelnen Klemmkörpern hängen von der gewünschten Belastbarkeit der Klemmverbindung zwischen Doppelstegplatte und Profil ab. Nach praktischen Versuchen ist es in der Regel ausreichend, die Klemmkörper in verhältnismäßig großen Abständen, wie etwa alle 7-10 Luftkammern, anzubringen.

Die bei dem erfindungsgemäßen Doppelstegplattenelement zu verwendenden Profile können je nach Wunsch aus Kunststoff oder aus Metall bestehen. Die Klemmkörper wird man zweckmäßig aus Kunststoff herstellen.

In Fig. 7 ist gezeigt, wie ein Aneinanderreihen von Doppelstegplattenelementen mit Hilfe von Anschlußprofilen nach Fig. 2 unter Verwendung eines zusätzlichen Verbindungsprofiles 8 möglich ist. Fig. 8 zeigt eine Eck- bzw. Scharnierverbindung von Doppelstegplattenelementen mit Anschlußprofilen nach Fig. 5.

In Fig. 9 schließlich ist ein Anschlußprofil 9 zum direkten Aneinanderreihen zweier Doppelstegplatten dargestellt.

Es versteht sich, daß sich die Erfindung nicht auf die Anwendung bei Frühbeetkästen oder Kleingewächshäusern beschränkt, sondern überall dort, wo Doppelstegplatten Verwendung finden, zum Einsatz kommen kann, wie beispielsweise bei aus Doppelstegplatten aufgebauten Fensterkonstruktionen, Lichtkuppeln, Lichtbändern und ähnlichen.

Obwohl in sämtichen dargestellten Ausführungsbeispielen die Klemmkörper an zwei einander gegenüberliegenden Seiten einen Vorsprung aufweisen und die Profile jeweils zwei einander gegenüberliegende Nasen besitzen, ist selbstverständlich auch eine Lösung mit nur einem Vorsprung der Klemmkörper und nur einer Nase des Profils denkbar. Ebenso ist es nicht unbedingt erforderlich, daß die Abmessung der Klemmkörper auf Höhe der Vorsprünge die lichte Weite zwischen den Deckplatten der Doppelstegplatte übersteigt und dadurch letztere im Bereich der Vorsprünge aufgeweitet wird. Es ist ohne weiters denkbar, die Abmessung der Klemmkörper auf Höhe der Vorsprünge gleich der lichten Weite zwischen den Deckplatten der Doppelstegplatte zu wählen, in welchem Fall dann die Doppelstegplatte hinter den Vorsprüngen der Klemmkörper durch die Nasen des Profils zusammengedrückt wird.

## Patentansprüche

1. Doppelstegplattenelement (1) mit von Anschluß- bzw. Abschlußprofilen (2) eingefaßten Rändern, insbesondere für Frühbeetkästen oder Kleingewächshäusern, dadurch gekennzeichnet, daß jeweils am Rand der Doppelstegplatte (1) Klemmkörper (3) in die von den Deckplatten (1a) und den Stegwänden (1b) der Doppelstegplatte (1) gebildeten Hohlkammern, oder durch Ausnehmungen (7) in den Stegwänden (1b) hindurch, im Abstand von einander eingesetzt sind, daß jeder Klemmkörper (3) zumindest einen, einer der beiden Deckplatten (1a) zugewandten Vorsprung (4) besitzt und daß das den jeweiligen Rand der Doppelstegplatte (1) umgreifende Anschluß- bzw. Abschlußprofil (2) für jeden Klemmkörper (3) jeweils zumindest eine, dem Vorsprung (4) des Klemmkörpers (3) zugeordnete, nach innen vorspringende Nase (5) aufweist, die zusammen mit der jeweiligen Deckplatte (1a) hinter dem Vorsprung (4) des jeweiligen Klemmkörpers eingerastet ist.

2. Doppelstegplattenelement nach Anspruch 1, dadurch gekennzeichnet, daß jeder Klemmkörper (3) an zwei gegenüberliegenden, den beiden Deckplatten (1a) zugewandten Seiten mit Vorsprüngen (4) versehen ist, die mit den Innenseiten der Deckplatten (1a) in Eingriff stehen, und daß das jeweilige Anschluß- bzw. Abschlußprofil (2) für jeden Klemmkörper (3) einander gegenüberliegende, nach innen vorspringende Nasen (5) besitzt, die mit den Außenseiten der Deckplatten (1a) im Eingriff stehen, wobei jede Deckplatte (1a) jeweils zwischen einem Vorsprung (4) eines Klemmkörpers (3) und einer diesen hintergreifenden Nase (5) des Anschluß- bzw. Abschlußprofiles (2) eingeklemmt ist.

3. Doppelstegplattenelement nach Anspruch 1 oder 2, dadurch gekennzeichnet, daß die Abmessung der Klemmkörper (3) auf Höhe ihres Vorsprunges bzw. ihrer Vorsprünge (4) größer ist als die lichte Weite zwischen den beiden Deckplatten (1a) der Doppelstegplatte (1).

4. Doppelstegplattenelement nach einem der Ansprüche 1 bis 3, dadurch gekennzeichnet, daß die Vorsprünge (4) der Klemmkörper (3) ein sägezahnartiges Profil aufweisen.

5. Doppelstegplattenelement nach einem der Ansprüche 1 bis 4, dadurch gekennzeichnet, daß die Klemmkörper (3) an ihren dem Rand der Doppelstegplatte zugeordneten Enden mit Ansätzen (3a) versehen sind, die einen Begrenzungsanschlag gegen zu weites Eindrücken der Klemmkörper (3) in die Hohlkammer der Doppelstegplatte (1) bilden.

6. Doppelstegplattenelement nach einem der Ansprüche 1 bis 4, dadurch gekennzeichnet, daß die Klemmkörper (3) in einem vorbestimmten Abstand auf einem Trägerkörper (6) angebracht sind.

7. Doppelstegplattenelement nach einem der Ansprüche 1 bis 6, dadurch gekennzeichnet, daß der senkrecht zu den Deckplatten (1a) der Doppelstegplatte (1) liegende Längsschnitt eines Klemmkörpers (3) aus zwei mit ihren Basen aneinanderstoßenden Trapezen besteht, wobei die Vorsprünge (4) an der Stoßstelle der beiden Trapeze ausgebildet sind.

## Claims

1. A double-web panel element (1) comprising edges which are adjoined by profiled connecting and/or terminating elements (2), particularly for hotbed boxes or small hothouses, characterized in that spaced apart clamping members (3) have been inserted at each edge of the double-web panel (1) into the hollow chambers which are defined by the top plates (1a) and the web walls (1b) of the double-web panel (1) or through apertures (7) in the web walls (1b), each clamping member (3) has at least one projection (4), which faces one of the two top plates (1a), and the profiled connecting and/or terminating element (2) which is associated with each clamping member (3) and embraces the associated edge of the double-web panel (1) has at least one inwardly protruding nose (5), which is associated with the projection (4) of the clamping member (3) and together with the associated top plate (1a) has snapped behind the projection (4) of the associated clamping member.

2. A double-web panel element according to claim 1, characterized in that each clamping member (3) is provided with projections (4) at two mutually opposite sides, which face the two top plates (1a) and engage the cover plates (1a) on their inside surfaces, the profiled connecting and/or terminating element (2) for each clamping member (3) has mutually opposite, inwardly protruding noses (5), which interengage with the outside surfaces of the top plates (1a), and each top plate (1a) is clamped between a projection (4) of a clamping member (3) and a nose (5) of the profiled connecting and/or terminating element (2), which nose engages that projection (4) on the rear.

3. A double-web panel element according to claim 1 or 2, characterized in that the extent of the clamping members (3) on the level of their projection or projections (4) exceeds the clearance between the two top plates (1a) of the double-web panel (1).

4. A double-web panel element according to any of claims 1 to 3, characterized in that the projections (4) of the clamping members (3) have a saw-tooth profile.

5. A double-web panel element according to any of claims 1 to 4, characterized in that the clamping members (3) are provided at those ends which are associated with the edge of the double-web panel with extensions (3a), which constitute a limiting stop against an excessive forcing of the clamping members (3) into the hollow chamber of the double-web panel (1).

6. A double-web panel element according to any of claims 1 to 4, characterized in that the clamping members (3) are mounted on a carrier member (6) and are spaced a predetermined distance apart.

7. A double-web panel element according to any of claims 1 to 6, characterized in that that longitudinal section of a clamping member (3) which extends at right angles to the top plates (1a) of the double-web panel (1) consists of two trapeziums, which have abutting bases, and the projections (4) are formed at the butt joint between the two trapeziums.

## Revendications

1. Panneau double (1) à traverses bordé de profilés (2) de liaison ou de terminaison, en particulier pour cultures sous châssis ou petites serres, caractérisé en ce qu'au bord concerné du panneau double (1) à traverses, des éléments de serrage (3) sont insérés à distance les uns des autres dans les cavités formées par les plaques extérieures (1a) et les parois (1b) de traverse du panneau double (1) ou à travers des évidements (7) ménagés dans les parois (1b) de traverse, en ce que chaque élément de serrage (3) présente au moins une saillie (4) orientée vers l'une des deux plaques extérieures (1a) et en ce que le profilé (2) de liaison ou de terminaison qui enserre le bord correspondant du panneau double (1) à traverses présente pour chaque élément de serrage (3) au moins un bec (5) saillant vers l'intérieur, associé à la saillie (4) de l'élément de blocage (3) et encliqueté sur la plaque extérieure (1a) correspondante derrière la saillie (4) de l'élément de serrage correspondant.

2. Panneau double à traverses selon la revendication 1, caractérisé en ce que chaque élément de serrage (3) est muni de saillies (4) sur deux faces opposées orientées vers les deux plaques extérieures (1a), ces saillies (4) étant en prise avec les faces intérieures des plaques extérieures (1a), et en ce que chaque profilé (2) de liaison ou de terminaison présente pour chaque élément de serrage (3) des becs (5) saillant vers l'intérieur et se faisant face, qui sont en prise avec les faces extérieures des plaques extérieures (1a), chaque plaque extérieure (1a) étant serrée entre une saillie (4) d'un corps de serrage (3) et un bec (5), en prise derrière ladite saillie (4), d'un profilé (2) de liaison ou de terminaison.

3. Panneau double à traverses selon l'une des revendications 1 ou 2, caractérisé en ce que la dimension de l'élément de serrage (3) au droit de sa ou de ses saillie(s) (4) est supérieure à l'écartement entre les deux plaques extérieures (1a) du

panneau double (1) à traverses.

4. Panneau double à traverses selon l'une des revendications 1 à 3, caractérisé en ce que les saillies (4) de l'élément de serrage (3) présentent un profil rappelant une dent de scie.

5. Panneau double à traverses selon l'une des revendications 1 à 4, caractérisé en ce que les éléments de serrage (3) sont munis, à leur extrémité associée au bord du panneau double à traverses, d'une pièce rapportée (3a) qui constitue une butée empêchant d'enfoncer trop profondément l'élément de serrage (3) dans la cavité du panneau double (1) à traverses.

6. Panneau double à traverses selon l'une des revendications 1 à 4, caractérisé en ce que las éléments de serrage (3) sont disposés selon un écartement prédéterminé sur un élément de support (6).

7. Panneau double à traverses selon l'une des revendications 1 à 6, caractérisé en ce que la section longitudinale d'un élément de serrage (3), faite perpendiculairement aux plaques extérieures (1a) du panneau double à traverses (1), consiste en deux trapèzes accolés par leurs bases, les saillies (4) étant formées à la jonction des deux trapèzes.

# Fig.1

# Fig. 2

# Fig. 3

# Fig. 4

Fig. 6

Fig. 9

Fig. 8

Fig. 5

Fig. 7

2